# FASCICULE DE BREVET EUROPEEN

(11) **EP 0 805 806 B1**
(45) Date de publication et mention de la délivrance du brevet: **15.12.1999**
(21) Numéro de dépôt: 96901832.4
(22) Date de dépôt: 23.01.1996
(51) Int. Cl.: C07D 305/14, A61K 31/335

(54) **PROCEDE DE PREPARATION DU TRIHYDRATE DU (2R,3S)-3-BENZOYLAMINO-2-HYDROXY-3-PHENYLPROPIONATE DE 4,10-DIACETOXY-2ALFA-BENZOYLOXY-BETA,20-EPOXY-BETA-DIHYDROXY-9-OXO-TAX-11-EN-BETA-YLE**
VERFAHREN ZUR HERSTELLUNG VON TRIHYDRAT VON 4,10-DIACETOXY-2ALFA-BENZOYLOXY-5-BETA,20-EPOXY-1,7BETA-DIHYDROXY-9-OXO-TAX-11-EN-13ALFA-YL ESTER VON (2R,35)-3-BENZOYLAMINO-2-HYDROXY-3-PHENYLPROPIONSÄURE
METHOD FOR PREPARING 4,10-DIACETOXY-2ALFA-BENZOYLOXY-5BETA,20-EPOXY-1,7BETA-DIHYDROXY-9-OXO-TAX-11-EN-13ALFA-YL (2R,3S)-3-BENZOYLAMINO-2-HYDROXY-3-PHENYLPROPIONATE TRIHYDRATE

(30) Priorité: 25.01.1995 FR 9500816
(43) Date de publication de la demande: 12.11.1997
(73) Titulaire: RHONE-POULENC RORER S.A., 92160 Antony (FR)
(72) Inventeur: AUTHELIN, Jean-René, F-91180 Saint-Germain-lès-Arpajon (FR); DIDIER, Eric, F-75013 Paris (FR); LEVEILLER, Franck, F-94320 Thiais (FR); TAILLEPIED, Isabelle, F-94700 Maisons Alfort (FR)
(74) Mandataire: Le Pennec, Magali
(86) Numéro de dépôt international: FR9600104
(87) Numéro de publication internationale: WO9622984

(56) Documents cités:
- WO-A-94/21622
- CHEMICAL ABSTRACTS, vol. 113, no. 75, 1990 Columbus, Ohio, US; abstract no. 142641k, GUERITTE-VOEGELEIN ET AL. 'STRUCTURE OF A SYNTHETIC TAXOL PRECURSOR.' page 723; & ACTA CRYSTALLOGR. SECT. C: CRYST. STRUCT. COMM., vol. C46, no. 5, 1990 ENGL., pages 781-784,

## Description

La présente invention concerne un procédé de préparation du trihydrate de (2R,3S)-3-benzoylamino-2-hydroxy-3-phénylpropionate de 4,10-diacétoxy-2α-benzoyl-oxy-5β,20-époxy-1,7β-dihydroxy-9-oxo-tax-11-èn-13α-yle et le trihydrate.

Le (2R,3S)-3-benzoylamino-2-hydroxy-3-phénylpropionate de 4,10-diacétoxy-2α-benzoyloxy-5β,20-époxy-1,7β-dihydroxy-9-oxo-tax-11-èn-13α-yle (ou paclitaxel) présente des propriétés anticancéreuses et antileucémiques remarquables.

Le (2R,3S)-3-benzoylamino-2-hydroxy-3-phénylpropionate de 4,10-diacétoxy-2α-benzoyloxy-5β,20-époxy-1,7β-dihydroxy-9-oxo-tax-11-èn-13α-yle peut être soit isolé à partir de l'écorce des ifs soit préparé à partir de la baccatine III ou de la 10-désacétyl-baccatine III selon les procédés qui sont décrits plus particulièrement dans les demandes de brevets européens EP 0 336 840 ou EP 0 400 971 ou dans la demande internationale PCT WO 94/07878.

Il a été trouvé que le trihydrate du (2R,3S)-3-benzoylamino-2-hydroxy-3-phénylpropionate de 4,10-diacétoxy-2α-benzoyloxy-5β,20-époxy-1,7β-dihydroxy-9-oxo-tax-11-èn-13α-yle présente une stabilité très nettement supérieure à celle du produit anhydre. La présente invention concerne le trihydrate du (2R,3S)-3-benzoylamino-2-hydroxy-3-phénylpropionate de 4,10-diacétoxy-2α-benzoyloxy-5β,20-époxy-1,7β-dihydroxy-9-oxo-tax-11-èn-13α-yle.

Selon l'invention, le trihydrate du (2R,3S)-3-benzoylamino-2-hydroxy-3-phénylpropionate de 4,10-diacétoxy-2α-benzoyloxy-5β,20-époxy-1,7β-dihydroxy-9-oxo-tax-11-èn-13α-yle peut être obtenu après cristallisation du (2R,3S)-3-benzoylamino-2-hydroxy-3-phénylpropionate de 4,10-diacétoxy-2α-benzoyloxy-5β,20-époxy-1,7β-dihydroxy-9-oxo-tax-11-èn-13α-yle dans un mélange d'eau et d'un alcool aliphatique contenant 1 à 3 atomes de carbone, suivi du séchage du produit obtenu sous pression réduite et ensuite d'un maintien dans une humidité relative supérieure à 20 % à une température voisine de 25°C.

Pour la mise en oeuvre du procédé selon l'invention, il peut être particulièrement avantageux
- de mettre en solution ou en suspension le (2R,3S)-3-benzoylamino-2-hydroxy-3-phénylpropionate de 4,10-diacétoxy-2α-benzoyloxy-5β,20-époxy-1,7β-dihydroxy-9-oxo-tax-11-èn-13α-yle dans un alcool aliphatique contenant 1 à 3 atomes de carbone,
- de traiter la solution ou la suspension par de l'eau contenant éventuellement une base minérale telle que l'hydrogénocarbonate de sodium,
- de séparer les cristaux obtenus, puis
- de les sécher sous pression réduite, puis
- de les maintenir éventuellement en atmosphère dont l'humidité relative est supérieure à 20 % à une température voisine de 25°C.

Généralement, le (2R,3S)-3-benzoylamino-2-hydroxy-3-phénylpropionate de 4,10-diacétoxy-2α-benzoyloxy-5β,20-époxy-1,7β-dihydroxy-9-oxo-tax-11-èn-13α-yle est dissous dans un excès de l'alcool aliphatique, de préference le méthanol. De préférence, la quantité d'alcool est comprise entre 6 et 12 parties en poids par rapport au (2R,3S)-3-benzoylamino-2-hydroxy-3-phénylpropionate de 4,10-diacétoxy-2α-benzoyloxy-5β,20-époxy-1,7β-dihydroxy-9-oxo-tax-11-èn-13α-yle mis en oeuvre.

Généralement, l'eau est ajoutée de telle façon que le rapport pondéral eau/alcool soit compris entre 3/1 et 1/3. L'eau ajoutée peut contenir jusqu'à 10 % (p/v) d'une base minérale telle que l'hydrogénocarbonate de sodium de façon que le pH du mélange réactionnel soit supérieur ou égal à 7, de préférence compris entre 7 et 8, avant la séparation des cristaux.

Le trihydrate de (2R,3S)-3-benzoylamino-2-hydroxy-3-phénylpropionate de 4,10-diacétoxy-2α-benzoyloxy-5β,20-époxy-1,7β-dihydroxy-9-oxo-tax-11-èn-13α-yle qui cristallise est séparé, de préférence par filtration ou centrifugation, puis séché. Le séchage est effectué sous pression réduite, comprise entre 1 et 7 kPa, à une température voisine de 40°C et le produit obtenu est éventuellement maintenu dans une atmosphère dont l'humidité relative est supérieure à 20 % et à une température comprise entre 0 et 60°C, de préférence voisine de 25°C.

Pour la mise en oeuvre du procédé, il peut être avantageux d'effectuer la cristallisation en présence d'acide ascorbique qui est ajouté lors de la dissolution ou de la mise en suspension du (2R,3S)-3-benzoylamino-2-hydroxy-3-phénylpropionate de 4,10-diacétoxy-2α-benzoyloxy-5β,20-époxy-1,7β-dihydroxy-9-oxo-tax-11-èn-13α-yle dans l'alcool. Il est possible d'utiliser jusqu'à 1 % en poids d'acide ascorbique.

Le trihydrate du (2R,3S)-3-benzoylamino-2-hydroxy-3-phénylpropionate de 4,10-diacétoxy-2α-benzoyloxy-5β,20-époxy-1,7β-dihydroxy-9-oxo-tax-11-èn-13α-yle a été étudié par analyses thermogravimétrique et calorimétrique différentielle et par diffraction par les rayons X.

Plus particulièrement, l'analyse thermogravimétrique montre une perte de masse entre 25 et 140°C voisine de 6 %, ce qui correspond à trois molécules d'eau pour une molécule de (2R,3S)-3-benzoylamino-2-hydroxy-3-phénylpropionate de 4,10-diacétoxy-2α-benzoyloxy-5β,20-époxy-1,7β-dihydroxy-9-oxo-tax-11-èn-13α-yle.

Pour la mise en oeuvre du prodédé selon l'invention, lorsque l'on utilise du paclitaxel semi-synthétique obtenu selon les procédés qui sont décrits par exemple dans les brevets européens EP 0 336 840 ou EP 0 400 971 ou dans la demande internationale PCT WO 94/07878 qui conduisent intermédiairement au paclitaxel dont les fonctions hydroxy sont protégées, il est possible d'opérer directement sur la solution ou sur la suspension du (2R,3S)-3-benzoylamino-2-hydroxy-3-phénylpropionate de 4,10-diacétoxy-2α-benzoyloxy-5β,20-époxy-1,7β-dihydroxy-9-oxo-tax-11-èn-13α-yle obtenu après élimination des groupements protecteurs des fonctions hydroxy du cycle taxane et de la chaîne latérale. Par exemple, en opérant dans les conditions de la demande internationale PCT WO 94/07878, on obtient intermédiairement le (4S,5R)-3-benzoyl-2-(4-méthoxy-phényl)-4-phényl-1,3-oxazolidine-5-carboxylate de 4,10-diacétoxy-2α-benzoyloxy-5β,20-époxy-1-hydroxy-7β-triéthylsilyloxy-9-oxo-tax-11-èn-13α-yle dont les groupements protecteurs peuvent être éliminés au moyen d'acide trifluoroacétique dans le méthanol.

Les exemples suivants illustrent la présente invention.

### EXEMPLE 1

Dans un réacteur à l'abri de la lumière, on introduit 5,014 g de (4S,5R)-3benzoyl-2-(4-méthoxyphényl)-4-phényl-1,3-oxazolidine-5-carboxylate de 4,10-diacétoxy-2α-benzoyloxy-5β,20-époxy-1-hydroxy-7β-triéthylsilyloxy-9-oxo-tax-11-èn-13α-yle dont le titre est de 98 % (4,52 mmoles) et 50 cm3 de méthanol. A la suspension blanche agitée, on ajoute rapidement 7 cm3 d'acide trifluoroacétique. La température monte au voisinage de 35°C. Après refroidissement à une température voisine de 5°C, on ajoute 110 cm3 d'une solution aqueuse d'hydrogénocarbonate de sodium à 6 % (p/v). Le pH est égal à 7. On sépare les cristaux par filtration sur verre fritté et les lave par 4 fois 15 cm3 d'un mélange méthanol-eau (30-70 en volumes). Après séchage sous pression réduite à 35°C, on obtient 3,676 g de (2R,3S)-3-benzoylamino-2-hydroxy-3-phénylpropionate de 4,10-diacétoxy-2α-benzoyloxy-5β,20-époxy-1,7β-dihydroxy-9-oxo-tax-11-èn-13α-yle titrant 93,1 % et contenant environ 4,8 % d'eau.

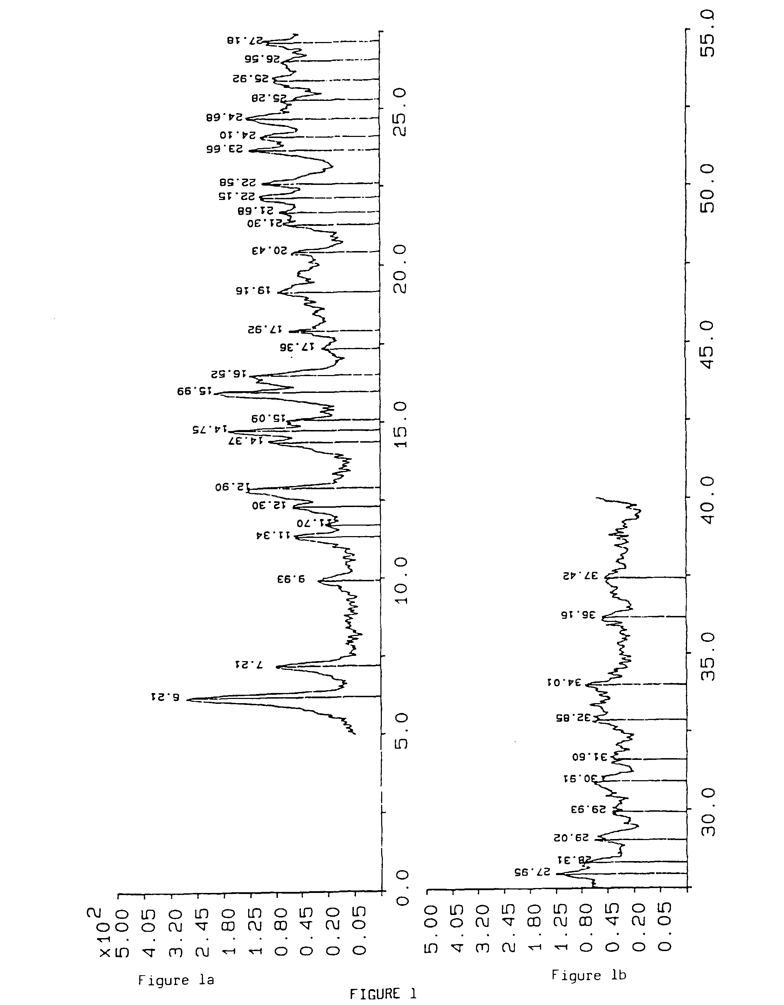

Le produit obtenu est caractérisé par le diagramme de poudre aux rayons X représenté par la figure 1.

Le rendement en produit pur est de 89,3 % par rapport à l'ester mis en oeuvre.

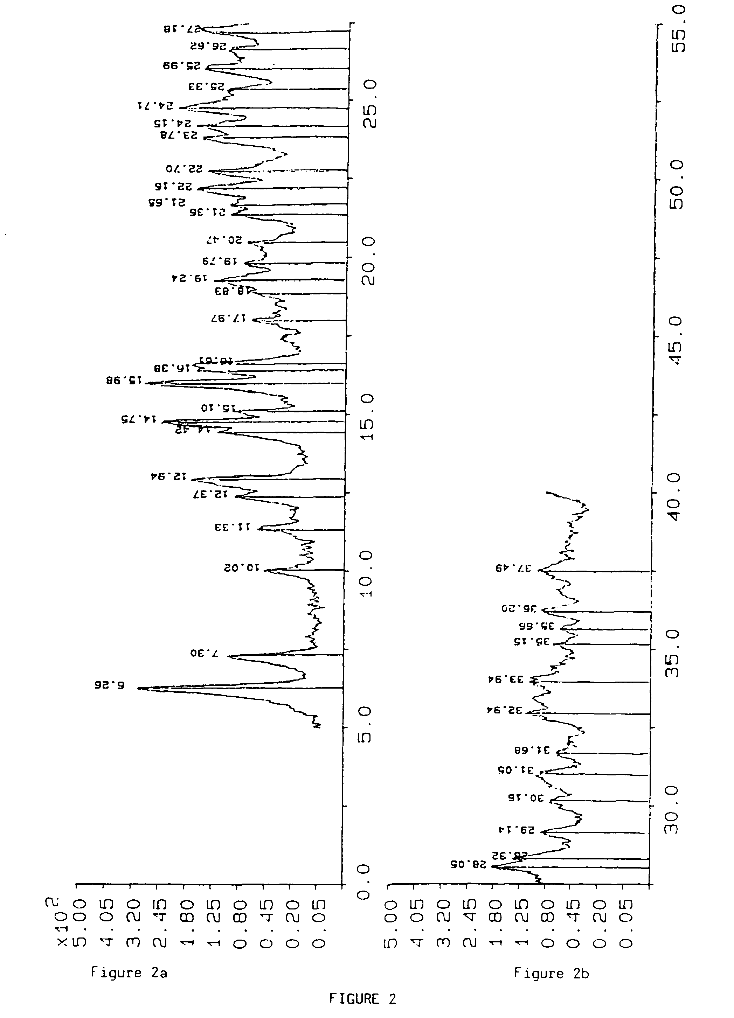

Maintenu dans des conditions d'humidité relative supérieure à 20 %, le produit se stabilise avec une teneur en eau voisine de 6 %. Le diagramme DPRX (diagramme de poudre aux rayons X), représenté par la figure 2, montre que le produit ainsi obtenu se présente sous forme d'un trihydrate (valeur théorique de la teneur en eau du trihydrate de (2R,3S)-3-benzoylamino-2-hydroxy-3-phénylpropionate de 4,10-diacétoxy-2α-benzoyl-oxy-5β,20-époxy-1,7β-dihydroxy-9-oxo-tax-11-èn-13α-yle de 5,95 %).

Le diagramme de poudre aux rayons X est réalisé au moyen d'un appareil Philips PW 1700® à tube anti-cathode de cobalt (λ K_{α1} = 1,7889 Å), le balayage étant effectué sous un angle de balayage initial de 5° 2-θ, de balayage final de 40° 2-θ, avec un pas de 0,02°0 2-θ à raison de 1 seconde par pas et en utilisant une pastille de silicium.

### EXEMPLE 2

Dans un réacteur à l'abri de la lumière, on introduit 3,006 g de (4S,5R)-3-benzoyl-2-(4-méthoxyphényl)-4-phényl-1,3-oxazolidine-5-carboxylate de 4,10-diacétoxy-2α-benzoyloxy-5β,20-époxy-1-hydroxy-7β-triéthylsilyloxy-9-oxo-tax-11-èn-13α-yle dont le titre est de 98 % (2,70 mmoles) et 30 cm3 de méthanol. A la suspension blanche agitée, on ajoute 6.3 cm3 d'acide trifluoroacétique à 99 %. Après refroidissement à une température voisine de 5°C, on ajoute 7,5 cm3 d'eau déminéralisée en 15 minutes. On sépare les cristaux par filtration sur verre fritté et les lave par 3 fois 5 cm3 d'un mélange méthanol-eau (80-20 en volumes) à 5°C. Après séchage sous pression réduite à 35°C, on obtient 1,989 g de (2R,3S)-3-benzoylamino-2-hydroxy-3-phénylpropionate de 4,10-diacétoxy-2α-benzoyloxy-5β,20-époxy-1,7β-dihydroxy-9-oxo-tax-11-èn-13α-yle titrant 97,8 % et contenant environ 6,8 % d'eau.

Le rendement est de 84,3 % par rapport à l'ester mis en oeuvre.

## Revendications

1. Procédé de préparation du trihydrate de (2R,3S)-3-benzoylamino-2-hydroxy-3-phénylpropionate de 4,10-diacétoxy-2a-benzoyloxy-5β,20-époxy-1,7β-dihydroxy-9-oxo-tax-11-èn-13α-yle caractérisé en ce que l'on cristallise le (2R,3S)-3-benzoylamino-2-hydroxy-3-phénylpropionate de 4,10-diacétoxy-2α-benzoyloxy-5β,20-époxy-1,7β-dihydroxy-9-oxo-tax-11-èn-13α-yle dans un mélange d'eau et d'un alcool aliphatique contenant 1 à 3 atomes de carbone, puis sèche du produit obtenu sous pression réduite puis le maintient éventuellement dans des conditions d'humidité relative supérieures à 20 %.

2. Procédé selon la revendication 1 caractérisé en ce que le rapport pondéral eau/alcool est compris entre 3/1 à 1/3.

3. Procédé selon l'une des revendications 1 ou 2 caractérisé en ce que l'alcool est le méthanol.

4. Procédé selon la revendication 1 caractérisé en ce que le séchage est effectué à un température voisine de 40°C sous pression réduite et que le produit se stabilise vers 6% d'eau dans une atmosphère dans laquelle l'humidité relative est supérieure à 20 %.

5. Procédé selon la revendication 1 caractérisé en ce que la cristallisation est effectuée en présence d'acide ascorbique.

6. Procédé selon la revendication 1 caractérisé en ce que l'on opère directement in situ sur l'ester résultant de l'estérification de la baccatine III dont la fonction hydroxy en -13 est protégée par un dérivé de la β-phénylisosérine protégé après élimination des groupements protecteurs.

7. Le trihydrate de (2R,3S)-3-benzoylamino-2-hydroxy-3-phénylpropionate de 4,10-diacétoxy-2α-benzoyloxy-5β,20-époxy-1,7β-dihydroxy-9-oxo-tax-11-èn-13α-yle.

## Patentansprüche

1. Verfahren zur Herstellung des Trihydrats von 4,10-Diacetoxy-2α-benzoyloxy-5β,20-epoxy-1,7β-dihydroxy-9-oxo-tax-11-en-13α-yl-Ester von (2R,3S)-3-Benzoylamino-2-hydroxy-3-phenylpropionsäure, dadurch gekennzeichnet, daß man den 4,10-Diacetoxy-2α-benzoyloxy-5β,20-epoxy-1,7β-dihydroxy-9-oxo-tax-11-en-13α-yl-Ester von (2R,3S)-3-Benzoylamino-2-hydroxy-3-phenylpropionsäure in einem Gemisch aus Wasser und einem aliphatischen Alkohol mit 1 bis 3 Kohlenstoffatomen kristallisiert, dann das erhaltene Produkt unter vermindertem Druck trocknet, es dann gegebenenfalls unter Bedingungen einer relativen Feuchtigkeit, die höher sind als 20%, hält.

2. Verfahren gemäß Anspruch 1, dadurch gekennzeichnet, daß das Gewichtsverhältnis Wasser/Alkohol zwischen 3/1 und 1/3 liegt.

3. Verfahren gemäß einem der Ansprüche 1 oder 2, dadurch gekennzeichnet, daß der Alkohol Methanol ist.

4. Verfahren gemäß Anspruch 1, dadurch gekennzeichnet, daß das Trocknen bei einer Temperatur nahe 40 °C unter vermindertem Druck ausgeführt wird und daß das Produkt in einer Atmosphäre, in der die relative Feuchtigkeit höher als 20% ist, sich in Richtung auf 6% Wasser stabilisiert.

5. Verfahren gemäß Anspruch 1, dadurch gekennzeichnet, daß die Kristallisation in Gegenwart von Ascorbinsäure ausgeführt wird.

6. Verfahren gemäß Anspruch 1, dadurch gekennzeichnet, daß man direkt in situ auf den Ester, der aus der Veresterung des Baccatin III hervorgeht, bei dem die Hydroxyfunktion in -13 durch ein geschütztes β-Phenylisoserin-Derivat geschützt wird, nach Eliminierung der Schutzgruppen einwirkt.

7. Das Trihydrat von 4,10-Diacetoxy-2α-benzoyloxy-5β,20-epoxy-1,7β-di-hydroxy-9-oxo-tax-11-en-13α-yl-Ester von (2R,3S)-3-Benzoylamino-2-hydroxy-3-phenylpropionsäure.

## Claims

1. Process for the preparation of 4,10-diacetoxy-2α-benzoyloxy-5β,20-epoxy-1,7β-dihydroxy-9-oxotax-11-en-13α-yl (2R,3S)-3-benzoylamino-2-hydroxy-3-phenylpropionate trihydrate, characterized in that 4,10-diacetoxy-2α-benzoyloxy-5β,20-epoxy-1,7β-dihydroxy-9-oxotax-11-en-13α-yl (2R,3S)-3-benzoylamino-2-hydroxy-3-phenylpropionate is crystallized from a mixture of water and an aliphatic alcohol containing 1 to 3 carbon atoms, then the product obtained is dried under reduced pressure and then optionally maintained under relative humidity conditions of greater than 20%.

2. Process according to claim 1, characterized in that the water/alcohol weight ratio is between 3/1 and 1/3.

3. Process according to either of claims 1 and 2, characterized in that the alcohol is methanol.

4. Process according to claim 1, characterized in that the drying is carried out at a temperature in the region of 40°C under reduced pressure and that the product stabilizes at about 6% water in an atmosphere in which the relative humidity is greater than 20%.

5. Process according to claim 1, characterized in that the crystallization is carried out in the presence of ascorbic acid.

6. Process according to claim 1, characterized in that the process is performed directly in situ on the ester resulting from the esterification of baccatin III, whose 13-hydroxy function is protected, with a protected β-phenylisoserine derivative after removal of the protecting groups.

7. 4,10-Diacetoxy-2α-benzoyloxy-5β,20-epoxy-1,7β-dihydroxy-9-oxotax-11-en-13α-yl (2R,3S)-3-benzoylamino-2-hydroxy-3-phenylpropionate trihydrate.
